# EUROPEAN PATENT SPECIFICATION

(11) **EP 2 042 077 B1**
(45) Date of publication and mention of the grant of the patent: **02.12.2009**
(21) Application number: 08015046.9
(22) Date of filing: 26.08.2008
(51) Int. Cl.: A61B 1/045

(54) **Electronic endoscope and endoscope system**
Elektronisches Endoskop und endoskopisches System
Endoscope électronique et système d'endoscope

(30) Priority: 26.09.2007 JP 2007248750
(43) Date of publication of application: 01.04.2009
(73) Proprietor: Fujinon Corporation, Saitama-shi, Saitama (JP)
(72) Inventor: Abe, Kazunori, Saitama-shi, Saitama (JP)
(74) Representative: Klunker . Schmitt-Nilson . Hirsch

(56) References cited:
- EP-A- 1 769 726
- US-A1- 2001 055 061
- US-A1- 2005 279 355

## Description

### BACKGROUND OF THE INVENTION

### 1. Technical Field

The invention relates to a wireless electronic endoscope that wirelessly transmits acquired imaging signals, and an endoscope system using the electronic endoscope.

### 2. Description of the Related Art

In the medical field, medical diagnosis is performed using an electronic endoscope. The electronic endoscope has an imaging device, such as a CCD, built in the tip end of an insertion part which is configured to be inserted into a body cavity. Imaging signals acquired by the CCD are converted into digital image data by various kinds of signal processing, and are then input to a processor device. The processor device further performs signal processing, etc. for the input image data, and then displays the result on a monitor. Thereby, we can observe images (endoscope images) within body cavities such as the stomach, intestines of a person being tested.

Generally, the electronic endoscope and the processor device are connected by a signal cable. In such an endoscope system, there is a problem that the signal cable might become obstructive when the electronic endoscope is operated, and/or that it might be inconvenient to carry the endoscope system. For this reason, a so-called wireless endoscope system that is configured to wirelessly transmit and receive signals between the electronic endoscope and the processor device has been proposed (for example, see JP 2001-231739 A (corresponding to U.S. Patent No.6,589,162)). Since the wireless endoscope system is good in operability and portability, the wireless endoscope system is preferably used, for example, for diagnosis at a bedside, in an emergency care field, etc..

The electronic endoscope for use in the wireless endoscope system cannot receive electric power from the outside via a power cable, etc. For this reason, a battery is used in the wireless electronic endoscope as a power source, and an operating time of the wireless electronic endoscope is limited. In this case, short operating time of the electronic endoscope becomes a cause which degrades maintenance properties, for example, replacement or charge of the battery should be performed frequently. Accordingly, in the wireless endoscope system, it is strongly demanded to prolong the life of the battery of the electronic endoscope as long as possible.

Furthermore, in the wireless electronic endoscope which uses the battery as a power source, battery exhaustion may unexpectedly occur during use. If the battery exhaustion occurs in a state where the insertion part is in the body cavity of a person being tested, an image cannot be confirmed when the insertion part is pulled out. Therefore, there is concern that the body cavity of the person being tested might be damaged by the tip end of the insertion part, etc.

As a measure which prevents such unexpected battery exhaustion, for example, it is conceivable that the remaining capacity of the battery is detected, and that if the remaining capacity becomes equal to or lower than a predetermined threshold, a warning is issued. In this case, if there is no margin for the threshold, the battery exhaustion might occur while the insertion part is being pulled out from the body cavity. Accordingly, some margin should be given to the threshold. However, giving excessive margin to the threshold might cause that the battery is determined as being exhausted while its capacity is left relatively much. As a result, there is a concern that the capacity of the battery cannot be efficiently utilized, and that the operating time of the electronic endoscope may be vainly shortened.

### SUMMARY OF THE INVENTION

The invention has been made in view of the above circumstances, prolongs the life of a battery of an electronic endoscope in a wireless endoscope system, and achieves a good balance between prevention of battery exhaustion during observation, and efficient utilization of the capacity of the battery.

According to an aspect of the invention, a wireless electronic endoscope includes an insertion part, an imaging device, a transmission section, a power circuit, a first modulation unit, a second modulation unit and a switching unit. The insertion part has a narrow tube shape and is configured to be inserted into an object being inspected. The imaging device is provided in a tip end of the insertion part and captures image light being focused on a light-receiving surface to acquire an imaging signal. The transmission section wirelessly transmits the imaging signal. The power circuit supplies electric power from a battery to respective sections. The first modulation unit modulates the imaging signal by a first modulation method. The second modulation unit that modulates the imaging signal by a second modulation method which consumes less electric power than the first modulation method. The switching unit selectively switches between the first modulation unit and the second modulation unit.

Also, the following configuration is preferable. That is, the electronic endoscope further includes a voltage detection unit that detects a voltage of the battery, and a voltage determination unit that determines as to whether or not the voltage is equal to or lower than a predetermined value. If the voltage determination unit determines that the voltage is equal to or lower than the predetermined value, the switching unit switches from the first modulation unit to the second modulation unit.

Also, it is preferable that the electronic endoscope further include an operation unit that arbitrarily selects one of the first and second modulation methods. It is more preferable that the first modulation method is an FM modulation method, and that the second modulation method is an SSB modulation method.

According to another aspect of the invention, an endoscope system includes a wireless electronic endoscope and a processor device. The wireless electronic endoscope includes an insertion part, an imaging device, a transmission section, a power circuit, a first modulation unit, a second modulation unit and a switching unit. The insertion part has a narrow tube shape and is configured to be inserted into an object being inspected. The imaging device is provided in a tip end of the insertion part and captures image light being focused on a light-receiving surface to acquire an imaging signal. The transmission section wirelessly transmits the imaging signal. The power circuit supplies electric power from a battery to respective sections. The first modulation unit modulates the imaging signal by a first modulation method. The second modulation unit modulates the imaging signal by a second modulation method which consumes less electric power than the first modulation method. The switching unit selectively switches between the first modulation unit and the second modulation unit. The processor device includes a reception section, a display unit, a first demodulation unit and a second demodulation unit. The reception section receives the imaging signal wirelessly transmitted from the electronic endoscope. The display unit displays the received imaging signal as an endoscope image. The first demodulation unit demodulates the imaging signal modulated by the first modulation method. The second demodulation unit demodulates the imaging signal modulated by the second modulation method.

Also, it is preferable that the processor device further includes a modulation method determining unit that determines which the first modulation method or the second modulation method the received imaging signal is modulated by, and inputs the received imaging signal to one of the first demodulation unit and the second demodulation unit based on a determination result.

Furthermore, it is more preferable that the processor device further includes a notification unit that notifies the modulation method by which the received imaging signal is modulated, based on the determination result of the modulation method determining unit.

In the invention, the electronic endoscope includes the first modulation unit that modulates the imaging signal by the first modulation method, the second modulation unit that modulates the imaging signal by the second modulation method, which consumes less power than the first modulation method, and the switching unit that selectively switches between the first modulation unit and the second modulation unit. Thereby, for example, by switching between the first and second modulation methods so that the second modulation method may be selected during insertion and pull-out of the insertion part and the first modulation method may be selected only during observation, electric power can be saved, and the life of the battery of the electronic endoscope can be prolonged.

Furthermore, by detecting the voltage of the battery, and if the voltage becomes equal to or lower than the predetermined value, then modulating the imaging signal by the second modulation method, which consumes less power, it becomes possible to set the threshold of the voltage to a value at which battery exhaustion may immediately occur if the first modulation method is kept used. Thus, the capacity of the battery can be efficiently utilized. Furthermore, the operating time of the electronic endoscope within the body cavity becomes long as power consumption decreases. As a result, it is possible to gain time enough to pull out the insertion part in the body cavity. Thus, the battery exhaustion during observation can be prevented.

### BRIEF DESCRIPTION OF THE DRAWINGS

Fig. 1 is an explanatory view schematically showing the configuration of an endoscope system.
Fig. 2 is a block diagram schematically showing the electric configuration of the electronic endoscope.
Fig. 3 is a graph showing an example of discharge curves of a battery when the electronic endoscope is driven.
Fig. 4 is a block diagram schematically showing the electric configuration of a processor device.
Figs. 5A and 5B are explanatory views showing examples of an endoscope image.

### DETAILED DESCRIPTION OF EMBODIMENTS OF THE INVENTION

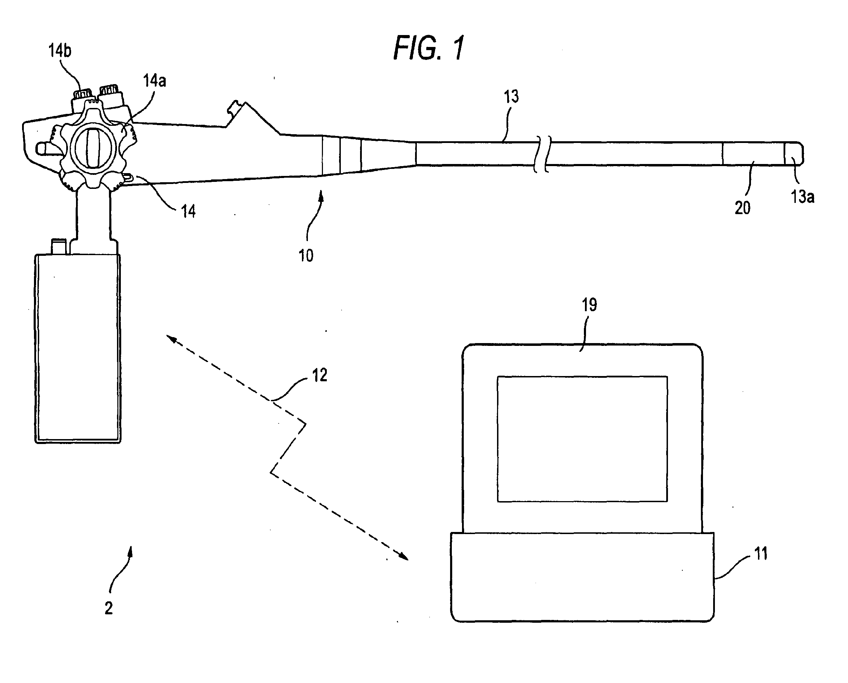

As shown in Fig. 1, an endoscope system 2 includes an electronic endoscope 10 and a processor device 11. The endoscope system 2 is a so-called wireless endoscope system which exchanges a signal between the electronic endoscope 10 and the processor device 11 by an electric wave 12.

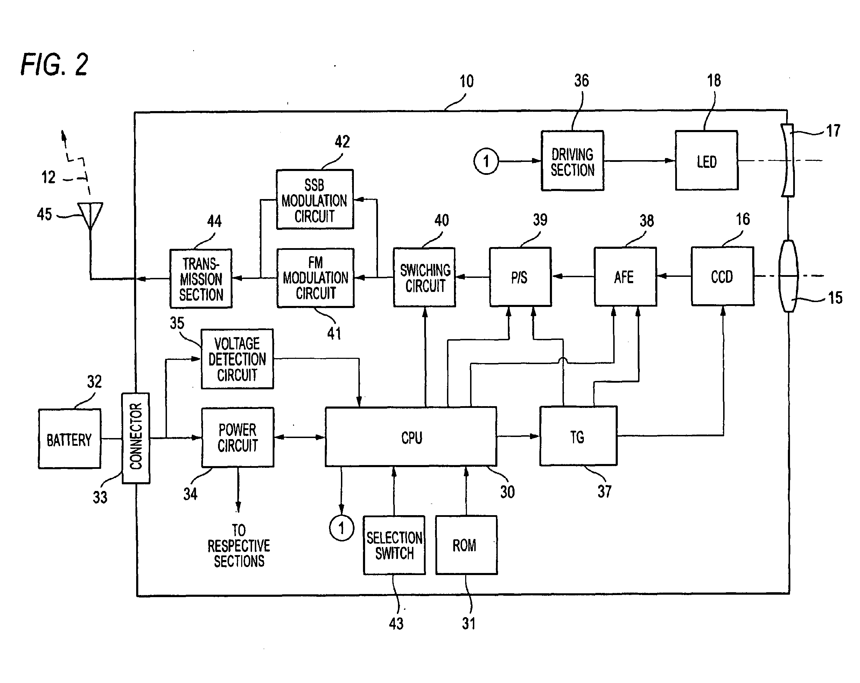

The electronic endoscope 10 includes an insertion part 13, which has a narrow tube shape and is configured to be inserted into a body cavity, and an operating part 14 connected to a base end of the insertion part 13. The tip end part 13a of the insertion part 13 includes therein an objective lens 15 for captures image light of an image of an observed object in a body cavity, a CCD 16 (for example, pixel size: 1280 x 960, frame rate: 30 frames/second) as an imaging device which images an observed body within the body cavity, an illumination lens 17, and an LED 18 for illuminate an inside of the body cavity (see Fig. 2 about all of them). An image within the body cavity acquired by the CCD 16 is displayed as an endoscope image on a monitor 19 (which may serve as a display unit and/or a notification unit) provided in the processor device 11.

A curved part 20 which is formed by connecting a plurality of curved pieces is provided behind the tip end part 13a. When an angle knob 14a provided in the operating part 14 is operated to push and pull a wire being inserted in the insertion part 13, the curved part 20 is operated so as to be curved vertically and horizontally, and the tip end part 13a is directed in a desired direction in the body cavity.

Fig. 2 is a block diagram schematically showing the electric configuration of the electronic endoscope 10. The whole operation of the electronic endoscope 10 is integrally controlled by a CPU 30 (which may serve as a voltage determination unit). A ROM 31 for storing various programs and data for controlling the operation of the electronic endoscope 10 is connected to the CPU 30. The CPU 30 reads a required program or data from the ROM 31, and then controls the operation of the electronic endoscope 10.

In the electronic endoscope 10A, a battery housing chamber (which is omitted in the figure) that houses a battery 32 is provided in a rear portion of the operating part 14. For example, a nickel hydrogen secondary battery is used as the battery 32. A connector 33 that contacts an electrode of the battery 32 is provided inside the battery housing chamber. The battery 32 housed in the battery housing chamber is connected to a power circuit 34 and a voltage detection circuit 35, which may serve as a voltage detection unit, via the connector 33. Also, the battery 32 is not limited to the nickel hydrogen secondary battery. For example, the battery 32 may be another secondary battery such as a lithium-ion secondary battery. Furthermore, the battery 32 is not limited to the secondary battery, and may be an alkali primary battery or a manganese primary battery.

The power circuit 34 increases or decreases the voltage of the battery 32 to a predetermined value and supplies the increased or decreased voltage to the respective components of the electronic endoscope 10. Thereby, the respective components such as the CPU 30 are activated. Furthermore, the power circuit 34 is connected to the CPU 30 via a signal line separately from a power source. The power circuit 34 individually switches ON/OFF of the electric power to be supplied to the respective components, based on a control signal from the CPU 30. The voltage detection circuit 35 detects the voltage of the battery 32, and inputs the detection result to the CPU 30.

A driving section 36 is connected to the LED 18. The driving section 36 drives ON/OFF of the LED 18 under the control of the CPU 30. Light emitted from the LED 18 is irradiated to an observed object in a body cavity via the illumination lens 17. Also, the following configuration may be adopted. That is, the LED 18 is arranged not in the tip end part 13a but inside the operating part 14, and the light is guided to the tip end part 13a by a light guide.

A timing generator (TG) 37 is connected to the CPU 30. The TG 37 is connected to the CCD 16, and to an AFE (Analog Front End)38 and a parallel/serial conversion unit (P/S) 39 which will be described later. The TG37 transmits a timing signal (a clock pulse) thereto. The CCD 16, the AFE 38, and the P/S 39 operate based on the timing signal transmitted from the TG 37.

Image light of an observed image of a body cavity which is incident through the objective lens 15 is focused onto an imaging surface of the CCD 16, and the CCD 16 outputs imaging signals corresponding to the image light from respective pixels. The AFE 38 performs correlated double sampling, amplification, and A/D conversion for the imaging signals input from the CCD 16, and then converts the imaging signals into digital image data. The P/S 39 converts the image data input from the AFE 38 from parallel data into serial data.

The P/S 39 is connected to a switching circuit 40. The switching circuit 40 switches a wiring path according to a control signal from the CPU 30, and selectively inputs the image data, which is the serial data obtained by the P/S 39, to either (i) an FM (Frequency Modulation) modulation circuit 41 which may serve as a first modulation unit or (ii) an SSB (Single Side Band) modulation circuit 42 which may serve as a second modulation unit. The CPU 30 and the switching circuit 40 may constitute a switching unit.

When image data is input via the switching circuit 40, the FM circuit 41 modulates the image data by an FM modulation method, and then generates a FM-modulated signal. When image data is input to the SSB modulation circuit 42 via the switching circuit 40, the SSB modulation circuit 42 modulates the image data by an SSB modulation method to generate an SSB-modulated signal.

Connected to the CPU 31 is a selection switch 43 (which may serve as an operation unit) for arbitrarily selecting the FM circuit 41 or the SSB modulation circuit 42. The selection switch 43 is, for example, a slide switch having a slider which is movable between a position where the FM modulation method is selected and a position where the SSB modulation method is selected. The selection switch 43 is provided in the operating part 14. The CPU 30 transmits a control signal to the switching circuit 40 according to the selection made by the selection switch 43, and then switches a wiring path so that modulation is performed by a selected modulation method.

Furthermore, the CPU 30 determines as to whether or not the voltage of the battery 32 is equal to or lower than a predetermined value, based on the detection result of the voltage of the battery 32 by the voltage detection circuit 35. Then, when the CPU 30 determines that the voltage is equal to or lower than the predetermined value, the CPU 30 transmits a control signal to the switching circuit 40, thereby instructing the switching circuit 40 to switch a wiring path so that modulation is performed by the SSB modulation circuit 42 irrespective of selection being made by the selection switch 43.

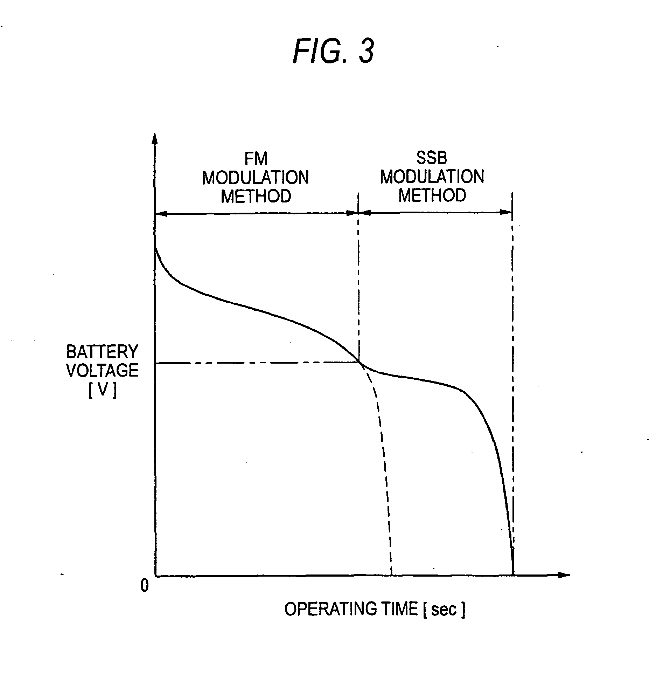

Fig. 3 is a graph showing an example of discharge curves of the battery 32 when the electronic endoscope 10 is driven. In Fig. 3, a solid line represents a discharge curve when switching from the FM modulation method to the SSB modulation method is performed in response to the fact that the voltage of the battery 32 becomes equal to or lower than the predetermined value. On the other hand, a dotted line represents a discharge curve when observation of an endoscope image is performed with the FM modulation method being kept.

The FM modulation method has little degradation associated with modulation and demodulation in resolution of an endoscope image as compared with the SSB modulation method, whereas it has higher power consumption than the SSB modulation method. In other words, the SSB modulation method has larger degradation associated with modulation and demodulation in resolution of an endoscope image as compared with the FM modulation method, whereas it has lower power consumption than the FM modulation method. For this reason, by forcibly switching to the SSB modulation method when the voltage (remaining capacity) of the battery 32 becomes equal to or lower than the predetermined value, the power consumption is suppressed, and as shown in Fig. 3, the operating time of the electronic endoscope 10 can be prolonged as compared with the case where observation is performed with the FM modulation method being kept.

Referring back to Fig. 2, the FM circuit 41 and the SSB modulation circuit 42 are connected to a transmission section 44. The transmission section 44 performs power amplification for the FM-modulated signal input from the FM circuit 41 or the SSB-modulated signal input from the SSB modulation circuit 42. Also, the transmission section 44 inputs the amplified modulation signal to an antenna 45, and wirelessly transmits the modulation signal to the processor device 11 as an electric wave 12.

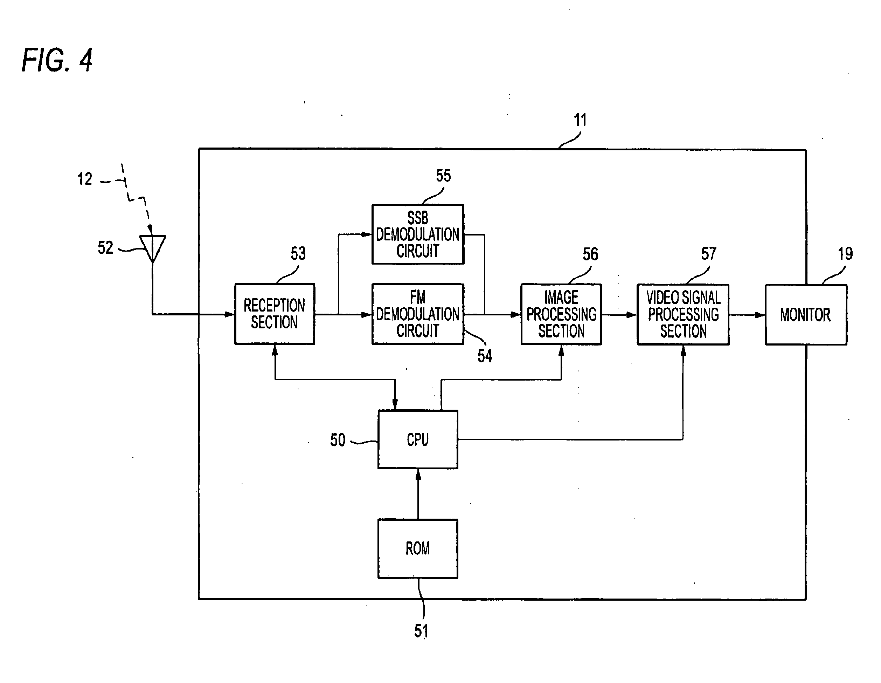

Fig. 4 is a block diagram schematically showing the electric configuration of the processor device 11. The whole operation of the processor device 11 is collectively controlled by the CPU 50. Connected to the CPU 50 is an ROM 51 that stores various programs and data for controlling the operation of the processor device 11. The CPU 50 reads a required program or data from the ROM 51, and then controls the operation of the processor device 11. The electric power of the processor device 11 may be supplied from a battery or may be supplied from the outside via a power cable, etc.

An antenna 52 receives the electric wave 12 from the electronic endoscope 10 and converts the received electric wave into an electric modulation signal. A reception section 53 (which may serve as a modulation method determining unit) amplifies the modulation signal received by the antenna 52, and determines, based on a wave form of the modulation signal and the like, as to whether or not the modulation signal received is a FM-modulated signal or an SSB-modulated signal. If the reception section 53 determines that the received modulation signal is a FM-modulated signal, the reception section 53 inputs the FM-modulated signal to an FM demodulation circuit 54 (which may serve as a first demodulation unit). If the reception section 53 determines that the received modulation signal is an SSB-modulated signal, the reception section 53 inputs the SSB-modulated signal to an SSB demodulation circuit 55 (which may serve as a second demodulation unit). Furthermore, the reception section 53 is connected to the CPU 50, and inputs a determination result regarding the modulation signal to the CPU 50.

The FM demodulation circuit 54 demodulates the FM-modulated signal input from the reception section 53 by an FM demodulation method to generate original image data from the FM-modulated signal. The SSB demodulation circuit 55 demodulates the SSB-modulated signal input from the reception section 53 by an SSB demodulation method to generate original image data from the SSB-modulated signal. The respective demodulation circuits 54 and 55 are connected to an image processing section 56. The image data demodulated by each of the demodulation circuits 54 and 55 is input to the image processing section 56.

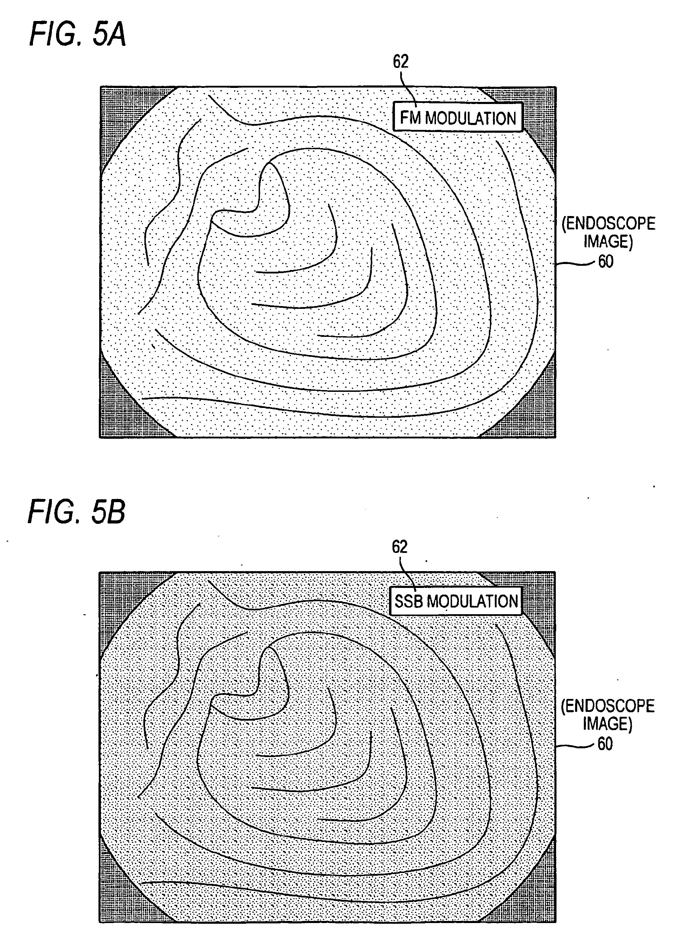

The image processing section 56 performs various kinds of image processing for the input image data. A video signal processing section 57 overlays masks or character information on the image data processed by the image processing section 56 under the control of the CPU 50, to thereby generate an endoscope image 60 shown in Fig. 5. Also, the video signal processing section 57 displays the generated endoscope image 60 on a monitor 19.

At the upper right of the endoscope image 60, modulation method information 62 which indicates a type of a modulation signal determined by the reception section 53 is displayed as character information. In the case where the received modulation signal is a FM-modulated signal, as shown in Fig. 5A, the video signal processing section 57 displays "FM modulation" as the modulation method information 62. On the other hand, in the case where the received modulation signal is an SSB-modulated signal, as shown in Fig. 5B, the video signal processing section displays "SSB modulation" as the modulation method information 62. As such, the processor device 11 overlays and displays the modulation method information 62 on the endoscope image 60, thereby notifying an operator, etc. of the modulation method of the received image data.

Next, the operation of the endoscope system 2 having the above configuration will be described. When the inside of the body cavity of a subject is observed by the endoscope system 2, first, a power switch (which is omitted in the figure) of the electronic endoscope 10 is turned on. Also, when the inside of the body cavity is observed, the selection switch 43 is operated to select the FM modulation method having little degradation in resolution. When the power switch is turned on, the electric power of the battery 32 is supplied to the respective components by the power circuit 34, thereby activating the CPU 30, etc.

The activated CPU 30 transmits a control signal to the driving section 36, thereby turning on the LED 18. Simultaneously, the CPU 30 transmits a control signal to the switching circuit 40 to switch a wiring path so that image data is input to the FM circuit 41. Furthermore, when the power source is turned on, the CPU 30 receives a detection result of the voltage of the battery 32 from the voltage detection circuit 35, and then determines as to whether or not the voltage of the battery 32 is equal to or lower than the predetermined value.

The image light incident from the objective lens 15 is focused on the imaging surface of the CCD 16, and imaging signals are output from the CCD 16. The imaging signals output from the CCD 16 are subjected to the correlated double sampling, the amplification, and the A/D conversion in the AFE 38, and are then converted into digital image data. The digital image data output from the AFE 38 is converted into image data of serial data by the P/S 39. The image data output from the P/S 39 is input to the FM circuit 41 via the switching circuit 40, and is converted into a FM-modulated signal. The FM-modulated signal is amplified by the transmission section 44, and is wirelessly transmitted as the electric wave 12 from the antenna 45.

The processor device 11 receives the FM-modulated signal transmitted as the electric wave 12 from the electronic endoscope 10 by the antenna 52. The received FM-modulated signal is amplified in the reception section 53. Furthermore, the reception section 53 determines a type of the received modulation signal based on a waveform of the modulation signal and the like. If the reception section 53 determines that the received modulation signal is a FM-modulated signal, the reception section 53 inputs the amplified FM-modulated signal to the FM demodulation circuit 54, and inputs to the CPU 50 the fact that the received modulation signal is the FM-modulated signal.

The FM-modulated signal input to the FM demodulation circuit 54 is demodulated to original image data by the FM demodulation circuit 54. The demodulated image data is subjected to various kinds of image processing in the image processing section 56. The image data output from the image processing section 56 is input to the video signal processing section 57. The video signal processing section 57 overlays and displays the modulation method information 62 of "FM modulation" on the input image data (see Fig. 5A), to generate the endoscope image 60. The generated endoscope image 60 is displayed on the monitor 19.

An operator inserts the insertion part 13 into the body cavity of a person being tested while observing the endoscope image 60 displayed on the monitor 19, thereby observing the inside of the body cavity. In this case, the operator can see the modulation method information 62, and then confirm that the modulation signal has been wirelessly transmitted by the FM modulation method according to the selection which is made with the selection switch 43, thereby knowing that the capacity of the battery 32 of the electronic endoscope 10 is left sufficiently.

If the CPU 30 of the electronic endoscope 10 determines that the voltage of the battery 32 becomes equal to or lower than the predetermined value based on the detection result from the voltage detection circuit 35, the CPU 30 transmits a control signal to the switching circuit 40 so that image data is input to the SSB modulation circuit 42. As such, by switching to the SSB modulation method with little power consumption, as shown in Fig. 3, the operating time of the electronic endoscope 10 can be prolonged. Thereby, even if the threshold of the voltage is set to a value where battery exhaustion immediately occur if the FM modulation method is kept, it is possible to gain time enough to pull out the insertion part 13 in the body cavity. Thus, battery exhaustion during observation can be prevented. In this case, there is a concern about degradation in resolution by the SSB modulation method. During insertion or pull-out of the insertion part 13, however, the endoscope image 60 is just confirmed unlike the case where an affected area, etc. is observed in detail. Thus, there is no concern that the degradation in resolution has a serious adverse effect on the insertion or pull-out of the insertion part 13.

Also, since the need for giving a superfluous margin to the threshold of a voltage is eliminated, it is possible to utilize the capacity of the battery 32 efficiently, and the operating time of the electronic endoscope 10 can be prevented from being shortened. Furthermore, modulation methods may be switched by the selection switch 43 so that the SSB modulation method is selected during the insertion or pull-out of the insertion part 13 and the FM modulation method is selected only during observation. Thereby, power consumption during the insertion or pull-out is suppressed, and the battery 32 can enjoy a long life.

If the processor device 11 receives the SSB-modulated signal from the electronic endoscope 10, the processor device 11 switches display of the modulation method information 62 from "FM modulation" to "SSB modulation" based on the determination result of the reception section 53 (see Fig. 5B). In the case where it is determined that the voltage of the battery 32 is equal to or lower than the predetermined value, and then switching to the SSB modulation method is performed, the display of the modulation method information 62 is switched although the selection switch 43 is not operated. Thus, this can make an operator, etc. immediately recognize that the battery 32 is exhausted.

Also, in the above embodiment, the modulation method information 62 notifies a modulation method. However, the invention is not limited thereto. For example, the warning relating to the fact that the battery 32 is exhausted may be directly displayed by transmitting a signal showing the fact that the battery 32 is exhausted along with image data from the electronic endoscope 10. Furthermore, in the above embodiment, notification is made by overlaying and displaying the modulation method information 62 on the endoscope image 60. However, the method of the notification, for example, may be performed by display lamps, such as an LED, or may be performed by voices, etc.

Meanwhile, the FM modulation method and the SSB modulation method are different in degree of the degradation of resolution. Therefore, the quality of the endoscope image 60 varies. Accordingly, an operator, etc. may recognize a modulation method from a difference in image quality without a notification unit or the like being provided separately.

Furthermore, in the above embodiment, the analog modulation methods such as the FM modulation method and the SSB modulation method are shown. However, the invention is not limited thereto. For example, digital modulation methods, such as a QAM (Quadrature Amplitude Modulation) modulation method, may be used. Also, when the digital modulation methods are adopted, the same effects as the above embodiment can be obtained even by changing the bit number of one modulation method, for example, when a 256-QAM modulation method is used as a first modulation method and a 16-QAM modulation method is used as a second modulation method. When the bit number has been changed in this way, the processor device 11 performs demodulation just by the QAM demodulation method regardless of a transmitted bit number. Thereby, since only one demodulation circuit is required, the configuration becomes simple, and the cost reduction, etc. of the endoscope system 2 can be achieved. Also, it should be noted that the respective analog and digital modulation methods are limited thereto. Any other modulation methods may be adopted so long as they have different power consumption, and are different in degree of degradation of resolution.

Moreover, in the above embodiment, modulation is performed only by one of the modulation circuits 41 and 42 by switching a wiring path. However, the invention is not limited thereto. For example, modulation may be performed only by one of the modulation circuits 41 and 42, by turning on/off the electric power to be supplied to the modulation circuits 41 and 42 and by driving only one of the modulation circuits 41 and 42 which perform modulation.

Also, in the above embodiment, the electronic endoscope for use in medical treatment for the body cavity of a person being tested as an object to be inspected is described as an example. However, the invention is not limited thereto. For example, the invention may be applied to an industrial electronic endoscope for a pipe as an object to be inspected.

## Claims

1. A wireless electronic endoscope (10) comprising:
an insertion part (13) that has a narrow tube shape and is configured to be inserted into an object being inspected;
an imaging device (15, 16) that is provided in a tip end (13a) of the insertion part and captures image light being focused on a light-receiving surface to acquire an imaging signal;
a transmission section (44) that wirelessly transmits the imaging signal;
a power circuit (34) that supplies electric power from a battery to respective sections;
a first modulation unit (41) that modulates the imaging signal by a first modulation method; **characterized in that** the endoscope further comprises
a second modulation unit (42) that modulates the imaging signal by a second modulation method which consumes less electric power than the first modulation method; and
a switching unit (40) that selectively switches between the first modulation unit and the second modulation unit.

2. The electronic endoscope according to claim 1, further comprising:
a voltage detection unit that detects a voltage of the battery, and
a voltage determination unit that determines as to whether or not the voltage is equal to or lower than a predetermined value, wherein
if the voltage determination unit determines that the voltage is equal to or lower than the predetermined value, the switching unit switches from the first modulation unit to the second modulation unit.

3. The electronic endoscope according to any one of claims 1 to 2, further comprising:
an operation unit that arbitrarily selects one of the first and second modulation methods.

4. The electronic endoscope according to any one of claims 1 to 3, wherein the first modulation method is an FM modulation method, and
the second modulation method is an SSB modulation method.

5. An endoscope system comprising:
the wireless electronic endoscope according to any one of claims 1 to 4; and
a processor device including
a reception section that receives the imaging signal wirelessly transmitted from the electronic endoscope,
a display unit that displays the received imaging signal as an endoscope image,
a first demodulation unit that demodulates the imaging signal modulated by the first modulation method, and
a second demodulation unit that demodulates the imaging signal modulated by the second modulation method.

6. The endoscope system according to claim 5, wherein the processor device further includes a modulation method determining unit that determines which the first modulation method or the second modulation method the received imaging signal is modulated by, and inputs the received imaging signal to one of the first demodulation unit and the second demodulation unit based on a determination result.

7. The endoscope system according to claim 6, wherein the processor device further includes a notification unit that notifies the modulation method by which the received imaging signal is modulated, based on the determination result of the modulation method determining unit.

## Patentansprüche

1. Drahtloses elektronisches Endoskop (10), umfassend:
einen Einführteil (13), der eine schmale schlauchförmige Form hat und zum Einführen in ein zu inspizierendes Objekt ausgebildet ist;
ein Abbildungs-Bauelement (15, 16), welches an einem freien Ende (13a) des Einführteils vorgesehen ist und zum Erfassen eines Abbildungssignals Bildlicht aufnimmt, welches auf eine Lichtempfangsfläche fokussiert wird;
einen Übertragungsabschnitt (44), der das Abbildungssignal drahtlos überträgt; eine Stromversorgungsschaltung (34), die elektrische Energie von einer Batterie zu einzelnen Abschnitten liefert;
eine erste Modulationseinheit (41), die das Abbildungssignal nach einem ersten Modulationsverfahren moduliert;
**dadurch gekennzeichnet, dass** das Endoskop weiterhin aufweist:
eine zweite Modulationseinheit (42), die das Abbildungssignal nach einem zweiten Modulationsverfahren moduliert, welches weniger elektrische Leistung verbraucht als das erste Modulationsverfahren; und
eine Umschalteinheit (40), die selektiv zwischen der ersten Modulationseinheit und der zweiten Modulationseinheit umschaltet.

2. Elektronisches Endoskop nach Anspruch 1, weiterhin umfassend:
eine Spannungsdetektoreinheit, die eine Batteriespannung ermittelt, und
eine Spannungs-Bestimmungseinheit, die feststellt, ob die Spannung gleich oder geringer ist als ein vorbestimmter Wert oder nicht, wobei
dann, wenn die Spannungs-Bestimmungseinheit feststellt, dass die Spannung gleich dem vorbestimmten Wert oder niedriger als der vorbestimmte Wert ist, die Umschalteinheit von der ersten Modulationseinheit auf die zweite Modulationseinheit umschaltet.

3. Elektronisches Endoskop nach einem der Ansprüche 1 bis 2, weiterhin umfassend:
eine Operationseinheit, die willkürlich eines von dem ersten und dem zweiten Modulationsverfahren auswählt.

4. Elektronisches Endoskop nach einem der Ansprüche 1 bis 3, bei dem das erste Modulationsverfahren ein FM-Modulationsverfahren und das zweite Modulationsverfahren ein ESB-Modulationsverfahren ist.

5. Endoskopsystem, umfassend:
das drahtlose elektronische Endoskop nach einem der Ansprüche 1 bis 4; und ein Prozessor-Bauelement, umfassend:
einen Empfangsteil, der das drahtlos von dem elektronischen Endoskop übertragene Abbildungssignal empfängt,
eine Anzeigeeinheit, die das empfangene Abbildungssignal als ein Endoskopbild darstellt,
eine erste Demodulationseinheit, die das nach dem ersten Modulationsverfahren modulierte Abbildungssignal demoduliert, und
eine zweite Demodulationseinheit, die das nach dem zweiten Modulationsverfahren modulierte Abbildungssignal demoduliert.

6. Endoskopsystem nach Anspruch 5, bei dem das Prozessor-Bauelement weiterhin eine Modulationsverfahren-Bestimmungseinheit aufweist, die ermittelt, nach welchem von dem ersten und dem zweiten Modulationsverfahren das empfangene Abbildungssignal moduliert wurde, und das empfangene Abbildungssignal basierend auf dem Ermittlungsergebnis an die erste Demodulationseinheit oder die zweite Demodulationseinheit gibt.

7. Endoskopsystem nach Anspruch 6, bei dem das Prozessor-Bauelement weiterhin eine Benachrichtigungseinheit aufweist, die basierend auf dem Ermittlungsergebnis der Modulationsverfahren-Bestimmungseinheit das Modulationsverfahren mitteilt, nach welchem das empfangene Abbildungssignal moduliert ist.

## Revendications

1. Endoscope électronique (10) sans fil comprenant :
un élément introductible (13) qui a la forme d'un tube étroit et est constitué pour être introduit dans un objet en cours d'examen ;
un dispositif (15, 16) de formation d'image qui est disposé à une extrémité (13a) de l'élément introductible et qui capte de la lumière d'image qui est focalisée sur une surface de réception de lumière pour acquérir un signal de formation d'image ;
une section (44) d'émission qui émet sans fil le signal de formation d'image ;
un circuit (34) d'alimentation qui fournit aux sections respectives de l'énergie électrique provenant d'une batterie ;
une première unité (41) de modulation qui module le signal de formation d'image par un premier procédé de modulation ;
**caractérisé en ce que** l'endoscope comprend en outre :
une seconde unité (42) de modulation qui module le signal de formation d'image par un second procédé de modulation qui consomme moins d'énergie électrique que le premier procédé de modulation., et
unité (40) de commutation qui commute sélectivement entre la première unité de modulation et la seconde unité de modulation.

2. Endoscope électronique selon la revendication 1, comprenant en outre :
une unité de détection de tension qui détecte la tension de la batterie ; et
une unité de détermination de tension qui détermine si la tension est ou non égale ou inférieure à une valeur prédéterminée,
dans lequel, si l'unité de détermination de tension détermine que la tension est égale ou inférieure à la valeur prédéterminée, l'unité de commutation passe de la première unité de modulation à la seconde unité de modulation.

3. Endoscope électronique selon l'une quelconque des revendications 1 et 2, comprenant en outre une unité de mise en oeuvre qui choisit arbitrairement l'un des premier et second procédés de modulation.

4. Endoscope électronique selon l'une quelconque des revendications 1 à 3,
dans lequel le premier procédé de modulation est un procédé de modulation à modulation de fréquence (FM), et
dans lequel le second procédé de modulation est un procédé de modulation à bande latérale unique (BLU).

5. Système d'endoscope comprenant :
l'endoscope électronique sans fil selon l'une quelconque des revendications 1 à 4 ; et
un dispositif processeur incluant :
une section de réception qui reçoit le signal de formation d'image émis sans fil par l'endoscope électronique ;
une unité d'affichage qui affiche le signal de formation d'image reçu sous forme d'une image d'endoscope ;
une première unité de démodulation qui démodule le signal de formation d'image modulé par le premier procédé de modulation ; et
une seconde unité de démodulation qui démodule le signal de formation d'image modulé par le second procédé de modulation.

6. Système d'endoscope selon la revendication 5, dans lequel le dispositif processeur inclut en outre une unité de détermination de procédé de modulation qui détermine par lequel du premier procédé de modulation ou du second procédé de modulation le signal de formation d'image est modulé, et qui entre le signal de formation d'image dans l'une de la première unité de démodulation et de la seconde unité de démodulation en se basant sur le résultat de détermination.

7. Système d'endoscope selon la revendication 6, dans lequel le dispositif processeur inclut en outre une unité de notification qui notifie le procédé de modulation par lequel le signal de formation d'image reçu est modulé, en se basant sur le résultat de détermination de l'unité de détermination de procédé de modulation.
